Europäisches Patentamt

European Patent Office

Office européen des brevets

(19)

(11) Publication number: **0 029 709**
**B1**

(12) **EUROPEAN PATENT SPECIFICATION**

(45) Date of publication of patent specification: **28.03.84**

(21) Application number: **80304157.3**

(22) Date of filing: **20.11.80**

(51) Int. Cl.³: **C 07 C 69/54,**
**C 07 C 67/26, B 01 J 23/26**

(54) Acrylate production.

(30) Priority: **02.04.80 US 136440**
**27.11.79 US 97932**

(43) Date of publication of application:
**03.06.81 Bulletin 81/22**

(45) Publication of the grant of the patent:
**28.03.84 Bulletin 84/13**

(84) Designated Contracting States:
**BE DE FR GB IT NL**

(56) References cited:
**FR - A - 1 473 715**
**GB - A - 1 195 485**
**US - A - 3 708 524**
**US - A - 4 017 429**

**Rollinson in Comprehensive Inorganic**
**Chemistry, Vol. 3, page 692, Pergamon Press**
**1973, 1975**

(73) Proprietor: **ICI AMERICAS INC**
**Concord Pike & New Murphy Road**
**Wilmington Delaware 19897 (US)**

(72) Inventor: **Markiewitz, Kenneth Helmuth**
**4012 Greenmount Road**
**Wilmington Delaware 19810 (US)**

(74) Representative: **Jones, Martin et al,**
**Imperial Chemical Industries PLC Legal**
**Department: Patents Po Box 6**
**Welwyn Garden City Herts, AL7 1HD (GB)**

Acrylate production

This invention concerns the production of hydroxy alkyl acrylates and methacrylates. More particularly, this invention relates to an improved method for the production of hydroxy alkyl acrylates and methacrylates by the chromium trioxide-catalyzed reaction of an alkylene oxide with acrylic or methacrylic acid.

In Steckler U.S. Patent 3,875,211 there is disclosed a method wherein a trivalent chromium salt is utilized as a catalyst in the reaction of acrylic acid and alkylene oxide. U.S. Patent 3,708,524 dated January 2, 1973 suggests the use of a chromium compound in combination with a trivalent iron compound as catalyst in the production of hydroxy alkyl acrylates or hydroxy alkyl methacrylates. See also U.S. patent 3,632,854. Japanese Patent Publication 97146 of 1975 describes the use of chromium trioxide as catalyst, but requires the presence of water or alcohol. None of the methods disclosed in the prior art, however, allow for the production of hydroxy alkyl methacrylates or acrylates under conditions which avoid a potential buildup of unreacted alkylene oxide while generating storage stable products of sufficient purity and freedom from deleterious amounts of catalysts. Such products allow them to be used directly as an intermediate in chemical processes such as for example, in polymerization processes for the manufacture of industrial resins.

It has now been discovered that the foregoing and related problems can be overcome by an improved process wherein the acrylic or methacrylic acid is initially mixed with an organic solvent in which the chromium trioxide catalyst is soluble followed by the sequential addition of alkylene oxide. The use of an oxide of chromium rather than any of the prior art chromium compounds, such as salts avoids the presence of foreign substances which may detrimentally affect the purity of the final product.

According to the invention there is therefore provided a process for the preparation of hydroxyalkyl acrylates or methacrylates by the reaction of an alkylene oxide with acrylic acid or methacrylic acid in the presence of at least 0.05% (based on the weight of the acid) of chromium trioxide which comprises the steps of

a) mixing the acid and chromium trioxide in a solvent;
b) adding the alkylene oxide to the solution in total amount of from 0.90 to 1.3 mole of alkylene oxide per mole of acrylic or methacrylic acid,

characterised in that (1) the solvent is a hydroxyalkyl acrylate or methacrylate in an amount of from 5 to 20% by weight of the acid, and in that (2) the reaction is carried out in the substantial absence of water.

The starting materials for the present invention are acrylic acid or methacrylic acid. An alkylene epoxide such as ethylene oxide, propylene oxide or butylene oxide is used as a coreactant. Normally the coreactants are used in about equal molar proportion. A preferred range suggested for the practice of the present invention is from 0.90 to 1.5 mols of alkylene oxide to 1 mol of the acrylic or methacrylic acid. A more preferred range is 1.00 to 1.2 mols of oxide to 1 mol of acid since a substantial excess of alkylene oxide tends to increase the formation of undesired byproducts.

The reaction is preferably conducted at a temperature within the range of 60° to 120°C and more preferably at a temperature 80° to 100°C. Lower temperatures provide for lower reaction rates, higher reaction temperatures tend to promote undesirable polymer formation. The reaction is normally run until the acid number of the reaction mixture has been brought below 30. An acid number of 5 to 15 indicates that the reaction has been essentially completed. Depending on the reaction temperature and the concentration of catalyst this will occur in from 20 to 1000 minutes. The longer times being necessary at lower temperatures and lower catalyst concentrations.

The catalyst to be employed in accordance with the present invention is chromium (VI) trioxide. The amount of catalyst utilized should be at least 0.05% by weight of acrylic or methacrylic acid. Naturally for economic reasons, the practitioner of the process should not utilize more catalyst than is necessary. Although it is possible to use as much catalyst as 5% by weight of acid, it should be recognized that as the amount of catalyst used is increased the resulting purity of hydroxy alkyl acrylate or methacrylate decreases. In those instances where the purity of the resulting product is not an issue, larger amounts of catalyst up to 5% may be desirable to achieve shorter reaction times. Final products having sufficient purity to allow their use without further purification for end uses such as the formation of unsaturated thermoset resins are obtained at catalyst levels of about .2% by weight of acid.

By premixing the acrylic or methacrylic acid, and the catalyst in an amount of solvent 5 to 20% by weight of acid prior to the addition of alkylene oxide, the process of the invention allows the catalyst to be most effective by providing an immediate reaction which begins upon the first addition of the alkylene oxide. It has been found that when weight of starting solvent is 15% of the amount of acid, the amount of alkylene oxide present during the reaction will not exceed a safe level of 7 to 10% by weight of total reaction mixture if the addition rate of alkylene oxide is about 0.5% by weight of total alkylene oxide per minute. At initial solvent levels of less than 5% the reaction proceed undesirably slowly, levels above 20% provide no advantages and reduce the economic advantage of the process. This suggested initial solvent level prevents the buildup of unreacted alkylene oxide which greatly minimizes exothermic conditions and risks of explosion.

Surprisingly it has also been discovered that the catalyst can be utilized in far smaller quantities than was heretofore known for reactions of alkylene oxide and acrylic or methacrylic acid. The ability of the process to function at such low catalyst levels provides for a surprisingly pure final product.

A small amount of a polymerization inhibitor is desirably incorporated into the reaction mixture to guard against acid and acrylate or methacrylate polymerization during the course of the reaction of the alkylene oxide with acrylic or methacrylic acid, particularly when the reaction is effected at slightly elevated temperatures such as above 130°C, which would otherwise result in contamination of the reaction product with acrylate or methacrylate polymer. A number of suitable polymerization inhibitors include: hydroquinone, monomethyl ether of hydroquinone, parabenzoquinone, t-butyl catechol and others. Two particular preferred inhibitor systems utilize a combination of t-butyl catechol and monomethyl ether of hydroquinone or parabenzoquinone and monomethylether of hydroquinone. The use of oxygen mixtures during the reaction, improves the effectiveness of the inhibitors allowing for extended reactor cycles.

The solvents that may be employed in the reaction may be any in which the chromium (VI) trioxide is soluble and which are miscible with alkylene oxide and acrylic methacrylic acid. Suitable solvents include tetrahydrofuran, dimethyl sulfoxide, higher boiling ketones such as, methyl isobutyl ketone and diethyl ketone, dimethyl formamide, hydroxy propyl methacrylate, hydroxy propyl acrylate among others. To avoid the need for further separation techniques it is an advantage to use the final product as the solvent for chromium trioxide. A preferred solvent in a method to prepare hydroxy propylmethacrylate would be hydroxy propyl methacrylate.

Should the final product be utilized in subsequent solvent polymerization reactions additional solvents such as styrene may provide economic advantages. The products of the present invention dissolved in styrene are suitable in the manufacture of unsaturated thermosetting urethane resins such as for example those disclosed in U.S. Patent 3,876,726 and 4,218,537.

Although the process of the present invention provides a product of sufficient purity to be immediately useful in further polymerization reactions, an even higher purity may be obtained by removing the chromium trioxide by for example a citric acid wash, a distillation or other well known purification techniques.

Since the presence of water in the hydroxy compounds is considered undesirable as it affects the utility of the hydroxy compound in subsequent polymerization reactions, precautions should be employed, such as vacuum stripping, to remove substantially all the water from the coreactants of the process.

The products of the process of the invention have been found to have excellent storage stability which is defined as the length of time the products prepared in accordance with the invention may be stored at 55°C before gellation occurs.

The present invention may be more fully understood in the following example which is offered by way of illustration. Parts by weight are used throughout unless otherwise indicated.

Example
Preparation of hydroxypropyl methacrylate

A vessel was charged 2.05 Kg of hydroxy propyl methacrylate having a molecular weight of 144.17, 13.64 Kg (158.1 gram mols) of methacrylic acid having a molecular weight of 86.09, 9.3 gms or 683 parts per million each of pure t-butyl catechol and hydroquinone monomethyl ether based on weight of MA charged and 17.7 gm or 1300 ppm of chromium trioxide based on MA. The mixture was heated to 90°C in a pressurized reactor and flushed with nitrogen followed by a evacuation. Final vacuum was released to provide an atmosphere of 5% oxygen in nitrogen to maintain the effectiveness of the inhibitor system. The methacrylic acid solution was added to the vessel where the solution was maintained at 90°C and 2.8 Kg/cm². A total of 9.6 Kg (165.6 gram mols) of propylene oxide was charged at the rate of 0.5% by weight of the total charge per minute for a total addition time of 200 minutes. The reaction continued till the acid number was approximately 15, after which the reaction mixture was cooled to 40°C. Although the reaction vessel of choice was for reasons of safety a vessel that could be pressured the reaction could be conducted at atmospheric pressures.

Excess propylene oxide may be removed by an aspirator vacuum. However, small amount of residual propylene oxide in the hydroxypropylmethacrylate have been found to be beneficial to the stability of saturated thermoset resins derived from it. The final product had a conversion yield of 98% based on methacrylic acid and was approximately 96% pure and could be used without further purification.

**Claims**

1. A process for the preparation of hydroxyalkyl acrylates or methacrylates by the reaction of an alkylene oxide with acrylic acid or methacrylic acid in the presence of at least 0.05% (based on the weight of the acid) of chromium trioxide which comprises the steps of

a) mixing the acid and chromium trioxide in a solvent;

b) adding the alkylene oxide to the solution in total amount of from 0.90 to 1.3 mole of alkylene oxide per mole of acrylic or methacrylic acid,

characterised in that 1. the solvent is a hydroxyalkyl acrylate or methacrylate in an amount of from 5 to 20% by weight of the acid, and in that 2. the reaction is carried out in the substantial absence of water.

2. A method as claimed in Claim 1 in which there is used 1.05 to 1.5 moles of alkylene oxide per mole of acrylic or methacrylic acid.

3. A method as claimed in Claim 1 or Claim 2 wherein the catalyst is present in an amount of 0.05 to 5% based on the weight of acid.

4. A method as claimed in Claim 1 or Claim 2 wherein the catalyst is present in an amount of 0.05 to 2% based on the weight of acid.

5. A method as claimed in Claim 1 or Claim 2 wherein the catalyst is present in an amount of 0.05 to 0.2% based on the weight of acid.

6. A method as in any one of Claims 1 to 5 wherein the solvent is previously obtained reaction product.

7. A method as claimed in any one of Claims 1 to 6 wherein the hydroxy alkyl methacrylate is hydroxy propyl methacrylate.

8. A method as claimed in any one of Claims 1 to 7 wherein a temperature range of 60° to 120°C is maintained during the reaction for a reaction time within the range of 1 to $4\frac{1}{2}$ hours.

9. A method as claimed in any one of Claims 1 to 8 wherein methacrylic acid and propylene oxide are reacted and the catalyst is present in a range of 0.10 to 1.4% based on the weight of the methacrylic acid.

**Patentansprüche**

1. Verfahren zur Herstellung von Hydroxyalkylacrylaten oder -methacrylaten durch Umsetzung eines Alkylenoxids mit Acrylsäure oder Methacrylsäure in Gegenwart von mindestens 0,05% (auf das Gewicht der Säure bezogen) Chromtrioxid, wobei das Verfahren die folgenden Schritte enthält:

a) Vermischen der Säure und des Chromtrioxids in einem Lösungsmittel,

b) Zugabe des Alkylenoxids zu der Lösung in einer Gesamtmenge von 0,90 bis 1,3 mol Alkylenoxid pro Mol Acryl- oder Methacrylsäure,

dadurch gekennzeichnet, daß (1.) das Lösungsmittel ein Hydroxyalkylacrylat oder -methacrylat in einer auf das Gewicht der Säure bezogenen Menge von 5 bis 20% ist und daß (2.) die Umsetzung im wesentlichen in Abwesenheit von Wasser durchgeführt wird.

2. Verfahren nach Anspruch 1, bei dem pro Mol Acryl- oder Methacrylsäure 1,05 bis 1,5 mol Alkylenoxid eingesetzt werden.

3. Verfahren nach Anspruch 1 oder 2, bei dem der Katalysator in einer Menge von 0,05 bis 5%, auf das Gewicht der Säure bezogen, vorliegt.

4. Verfahren nach Anspruch 1 oder 2, bei dem der Katalysator in einer Menge von 0,05 bis 2%, auf das Gewicht der Säure bezogen, vorliegt.

5. Verfahren nach Anspruch 1 oder 2, bei dem der Katalysator in einer Menge von 0,05 bis 0,2%, auf das Gewicht der Säure bezogen, vorliegt.

6. Verfahren nach einem der Ansprüche 1 bis 5, bei dem das Lösungsmittel ein vorher erhaltenes Reaktionsprodukt ist.

7. Verfahren nach einem der Ansprüche 1 bis 6, bei dem des Hydroxyalkylmethacrylat Hydroxypropylmethacrylat ist.

8. Verfahren nach einem der Ansprüche 1 bis 7, bei dem während der Umsetzung für eine Reaktionszeit in dem Bereich von 1 bis 4,5 h ein Temperaturbereich von 60° bis 120° Aufrechterhalten wird.

9. Verfahren nach einem der Ansprüche 1 bis 8, bei dem Methacrylsäure und Propylenoxid umgesetzt werden und der Katalysator in einem Bereich von 0,10 bis 1,4%, auf das Gewicht der Methacrylsäure bezogen, vorliegt.

**Revendications**

1. Procédé de préparation d'acrylates ou de méthacrylates d'hydroxyalcoyle par la réaction d'un oxyde d'alcoylène avec l'acide acrylique ou l'acide méthacrylique en présence d'au moins 0,05% (sur base du poids de l'acide) de trioxyde de chrome, suivant lequel:

a) on mélange l'acide et le trioxyde de chrome dans un solvant;

b) on ajoute l'oxyde d'alcoylène à la solution en une quantité totale de 0,90 à 1,3 mole d'oxyde d'alcoylène par mole d'acide acrylique ou méthacrylique,

caractérisé en ce que (1) le solvant est un acrylate ou méthacrylate d'hydroxyalcoyle en une quantité de 5 à 20%, du poids de l'acide et (2) la réaction est exécutée en l'absence sensible d'eau.

2. Procédé suivant la revendication 1, dans lequel on utilise 1,05 à 1,5 mole d'oxyde d'alcoylène par mole d'acide acrylique ou méthacrylique.

3. Procédé suivant la revendication 1 ou 2, dans lequel le catalyseur est présent en une quantité de 0,05 à 5%, sur la base du poids de l'acide.

4. Procédé suivant la revendication 1 ou 2, dans lequel le catalyseur est présent en une quantité de 0,05 à 2%, sur la base du poids de l'acide.

5. Procédé suivant la revendication 1 ou 2, dans lequel le catalyseur est présent en une quantité de 0,05 à 0,2%, sur la base du poids de l'acide.

6. Procédé suivant l'une quelconque des revendications 1 à 5, dans lequel le solvant est du produit de réaction obtenu antérieurement.

7. Procédé suivant l'une quelconque des revendications 1 à 6, dans lequel le méthacrylate d'hydroxyalcoyle est le méthacrylate d'hydroxypropyle.

8. Procédé suivant l'une quelconque des revendications 1 à 7, dans lequel une température de l'intervalle de 60 à 120°C est entretenue au cours de la réaction pendant une durée de réaction de l'intervalle de 1 à 4,5 heures.

9. Procédé suivant l'une quelconque des revendications 1 à 8, dans lequel l'acide méthacrylique et l'oxyde de propylène sont mis à réagir et le catalyseur est présent en quantité de l'intervalle de 0,10 à 1,4%, sur la base du poids de l'acide méthacrylique.